# EUROPÄISCHE PATENTSCHRIFT

(11) **EP 2 653 174 B1**
(45) Veröffentlichungstag und Bekanntmachung des Hinweises auf die Patenterteilung: **27.12.2017**
(21) Anmeldenummer: 13162943.8
(22) Anmeldetag: 09.04.2013
(51) Int. Cl.: A61L 31/08, A61L 31/14, A61L 31/10

(54) **IMPLANTAT UND VERFAHREN ZUR HERSTELLUNG DESSELBEN**
IMPLANT AND METHOD FOR MANUFACTURING SAME
IMPLANT ET SON PROCÉDÉ DE FABRICATION

(30) Priorität: 16.04.2012 US 201261624403 P
(43) Veröffentlichungstag der Anmeldung: 23.10.2013
(73) Patentinhaber: Biotronik AG, 8180 Bülach (CH)
(72) Erfinder: SCHMIEDL, Robert, 96114 Hirschaid (DE); GRATZ, Matthias, 91054 Erlangen (DE); SCHALDACH, Max, 12359 Berlin (DE); LANG, Volker, 12161 Berlin (DE)
(74) Vertreter: Galander, Marcus

(56) Entgegenhaltungen:
- WO-A1-2012/148265
- US-A1- 2004 077 948
- US-A1- 2005 074 406

## Beschreibung

Die Erfindung betrifft ein Verfahren zur Herstellung eines Implantats, insbesondere einer intraluminalen Endoprothese, mit einem Körper, der zumindest auf einem Teil seiner Oberfläche eine Beschichtung aufweist, sowie ein entsprechendes Implantat.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen einen Körper in der Form eines rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können.

Ein Ansatzpunkt zur Lösung dieses Problems besteht darin, den Stent bzw. andere Implantate aus einem degradierbaren oder biodegradierbaren Werkstoff zu fertigen.

Unter Degradation werden allgemein hydrolytische und oxidative Abbauprozesse verstanden, einschließlich metallkatalysierter Oxidation, während unter Biodegradation hydrolytische, enzymatische und andere Abbauprozesse im lebenden Organismus zusammengefasst werden, die vor allem durch die mit dem biodegradierbaren Werkstoff in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Es sind bereits Stents bekannt, die Beschichtungen mit verschiedenen Funktionen aufweisen. Derartige Beschichtungen dienen beispielsweise der Freisetzung von Medikamenten, der Anordnung eines Röntgenmarkers oder dem Schutz der darunter liegenden Strukturen. Ferner werden Beschichtungen zur Degradationssteuerung, beispielsweise zur Verzögerung der Degradation verwendet.

Bei der Realisierung biodegradierbarer Implantate soll die Degradierbarkeit entsprechend der angestrebten Therapie bzw. der Anwendung des jeweiligen Implantats (koronar, intracranial, renal etc.) gesteuert werden. Wünschenswert für viele therapeutische Anwendungen ist es, wenn das Implantat zu einem bestimmten, von außen vorgebbaren Zeitpunkt oder Zeitraum seine Integrität verliert oder beginnt zu verlieren. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammenfällt oder zusammenbricht, d. h. unter Belastung mindestens 80% des Dilatationsdurchmessers aufweist, oder im Fall eines Stents kaum angebrochene tragende Streben aufweist.
Im Stand der Technik sind bereits unterschiedliche Mechanismen der Degradationssteuerung von Implantaten beschrieben. Diese basieren beispielsweise auf anorganischen und organischen Schutzschichten oder deren Kombination, die dem Korrosionsmilieu im behandelten Organismus und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem Material des Implantatkörpers beruhen. Diese führen dazu, dass die Degradationszeit verlängert wird. So wird der Degradationsschutz durch verschieden zusammengesetzte Schutzschichten und durch definierte geometrische Abstände (Diffusionsbarrieren) zwischen dem Korrosionsmedium und dem Implantatkörpermaterial abgesichert. Andere Lösungen basieren darauf, Legierungsbestandteile des biodegradierbaren Materials des Implantatkörpers gezielt zu verändern. Mit den zuvor genannten Lösungen gelingt es jedoch nicht, die durch den Degradationsprozess eintretende Auflösung und die daraus resultierenden Stegbrüche in ein variables, jedoch von außen beispielsweise auf einen bestimmten Behandlungserfolg abgestimmtes Zeitfenster zu legen. Oft setzt bei Implantaten die Degradation zu früh oder zu spät ein bzw. die Dauer der Degradation des Implantats ist zu unbestimmt. EP2329853 offenbart eine intraluminale Endoprothese mit einem biodegradierbaren metallischen Implantatkörper und einer Beschichtung, die Strontium oder Calcium enthält, um damit eine Degradation des Implantats zu einem kontrollierbaren Zeitpunkt und in einem gewünschten Zeitfenster zu bewirken.

Ein weiteres Problem im Zusammenhang mit Beschichtungen und der Degradationssteuerung ergibt sich auch daraus, dass Stents oder andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert. Aufgrund dieser Durchmesseränderung ist der Körper des Implantats hierbei einer starken mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den oben genannten Beschichtungen ergibt sich somit der Nachteil, dass diese während der Verformung des Implantats reißt (z. B. Bildung von Mikrorissen) oder auch teilweise entfernt wird. Hierdurch kann eine lokale Degradation zu einem Zeitpunkt verursacht werden, zu dem eine Degradation noch nicht gewünscht ist. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Folglich besteht die Aufgabe der vorliegenden Erfindung darin, ein Implantat zu schaffen, das eine sichere Degradation des Implantats in einem variablen, von außen vorgebbaren Zeitfenster ermöglicht. Entsprechend besteht die Aufgabe der Erfindung außerdem darin, ein einfaches Verfahren zur Herstellung eines derartigen Implantats anzugeben.

Die obige Aufgabenstellung wird gelöst durch ein Verfahren umfassend die folgenden Schritte:
a) Bereitstellen des Implantatkörpers,
b) Aufbringen der Beschichtung auf die Oberfläche des Implantatkörpers, wobei die Beschichtung ungefüllte Hohlräume, vorzugsweise in Form von Microbubbles, aufweist.

Der Vorteil des dargestellten Verfahrens besteht darin, dass ein Implantat mittels eines einfachen Verfahrens hergestellt werden kann, wobei das Implantat eine von außerhalb des Körpers vorgebbare Degradationskontrolle (extrakorporale Degradationskontrolle) ermöglicht. Die ungefüllten und gegebenenfalls zusätzlich vorliegenden gefüllten Hohlräume schützen zunächst die Oberfläche des Implantatkörpers und unterbinden beziehungsweise minimieren die Degradation.

Im Sinne der vorliegenden Erfindung umfasst der Begriff "ungefüllte Hohlräume" solche Hohlräume oder Vesikel, die in dem Hohlraum ein Gas enthalten, jedoch nicht mit einer Flüssigkeit oder einem Feststoff gefüllt sind. Demgegenüber werden unter "gefüllten Hohlräumen" Hohlräume verstanden, die in ihrem Inneren einen Feststoff und/oder eine Flüssigkeit aufweisen.

Die vorliegende Erfindung beruht insbesondere auf der Erkenntnis, dass gasgefüllte Hohlräume kompressibel sind und daher im Sinne der vorliegenden Erfindung in eine Resonanz geraten können. (Dies trifft für flüssigkeits- oder feststoffgefüllte Vesikel nicht zu.) Aufgrund der Kompressibilität können ungefüllte Hohlräume zu einem beliebigen Zeitpunkt von einem Ort außerhalb des behandelten Organismus mittels Ultraschall zu Schwingungen angeregt werden, beispielsweise dann, wenn ein ausreichend weit fortgeschrittener Heilungsverlauf festgestellt wurde. Die mechanische Verformung der Wandungen der Hohlräume durch die Schwingungen aufgrund externer Intervention führt bei hinreichender Amplitude zum Bruch oder Reißen dieser Wandungen, so dass die Umhüllung der Hohlräume zerstört wird. Hierbei ist die zum Anregen der Schwingung benötigte Ultraschallamplitude umso niedriger, je näher die Frequenz der Anregungsschwingung der Resonanzfrequenz des anzuregenden Systems kommt. Bei Anregung mit der Resonanzfrequenz geraten die Hohlräume vor allen anderen Objekten der Umgebung in Schwingung.

Aufgrund der Zerstörung der Umhüllung (Wandungen) der Hohlräume wird die Degradation der Beschichtung beschleunigt, da hierdurch Teile der Beschichtung abgetrennt werden. Ferner wird ein besserer Zugang der in dem behandelten Organ vorliegenden Körperflüssigkeit zu tiefer liegenden Stellen des Implantats ermöglicht, so dass auch dort die Degradation voranschreitet. Die Zerstörung der Beschichtung kann dabei so weit gehen, dass die Oberfläche des Implantatkörpers zumindest teilweise freigelegt wird und dieser so unmittelbar degradiert.

Der Körper des Implantats umfasst mindestens einen Teil des Implantats, vorzugsweise den Hauptteil des Implantats, welcher die mechanische Integrität des Implantats bewirkt. Der Implantatkörper enthält in der vorliegenden Erfindung vorzugsweise ein Material oder mehrere Materialien aus der Gruppe enthaltend biodegradierbare Metalle wie Magnesium, Eisen, biodegradierbare Legierungen, aufweisend die Elemente Magnesium, Eisen, Zink, Molybdän und/oder Wolfram, biodegradierbare Polymere, z.B. Polylactide, Polylactid-co-Glycolide, Polyhydroxyalkanoate, Caprolactone, oder der Implantatkörper besteht aus einem Material oder mehreren Materialien dieser Gruppe.

In einem bevorzugten Ausführungsbeispiel werden die Hohlräume durch Microbubbles gebildet, welche auch als Bubbles oder Mikrobläschen bezeichnet werden. Microbubbles weisen eine flexible Umhüllung oder Hülle auf, während Mikrokapseln eine starre Schale haben. Der Vorteil der Verwendung von Microbubbles besteht darin, dass deren Umhüllung aufgrund ihrer Flexibilität die Fähigkeit zur resonanten Schwingung besitzt und demnach besonders effizient mittels Ultraschall zur Schwingung angeregt und zerstört werden kann. Dabei gibt es für eine erfindungsgemäße Beschichtung aus Microbubbles nur einen engen Frequenzbereich, in dem - bei fester Anregungsamplitude - der Amplitudengang der ungefüllten Bubbles auf ein Vielfaches seines Wertes bei niedrigeren oder höheren Frequenzen anwächst. Kennzeichnend für die Eigenschaft geeigneter Microbubbles ist eine mindestens zweifache Resonanzüberhöhung, bevorzugt mindestens dreifache, weiter bevorzugt mindestens fünffache Resonanzüberhöhung, in dem genannten engen Frequenzbereich gegenüber den Frequenzen, die z.B. im Rahmen diagnostischer Anwendung von Ultraschall Verwendung finden.

In der vorliegenden Erfindung enthalten ungefüllte Hohlräume z.B. Luft oder Stickstoff. Bevorzugt kann als Füllmedium ein Gas mit großen Molekülen verwendet werden, welche nicht durch die Wand des Hohlraums in die umgebende Körperflüssigkeit (z.B. Blut) diffundieren können und/oder welche eine geringe Löslichkeit in Blut aufweisen, z.B. Perfluorocarbone (z.B. Perfluorobutan n-C₄F₁₀) oder SF₆.

In einem Ausführungsbeispiel zusätzlich vorzusehende gefüllte Hohlräume weisen demgegenüber als Füllung ein flüssiges oder festes Material, beispielsweise eine flüssige oder feste pharmazeutisch aktive Substanz und/oder eine diagnostische Substanz, auf.

Es ist hinsichtlich der vorliegenden Erfindung weiter von Vorteil, dass die zur Degradation benutzte Ultraschallanregung gleichzeitig auch die Effizienz der Wirkstoffaufnahme in den Zellen des Zielgewebes der Behandlung, beispielsweise in den Zellen eines Blutgefäßes erhöht. Nach der Implantation befinden sich die Hohlräume, die durch die Ultraschallanregung zum Platzen gebracht werden können, in unmittelbarer Nachbarschaft zu Zellen des Zielgewebes. Die Zerstörung der Hohlräume durch Ultraschall wirkt durch die räumliche Nähe zum Zielgewebe auch in dem Zielgewebe sonoporierend. Dadurch können Moleküle einer pharmazeutisch aktiven Substanz nicht nur über die Transportmechanismen einer intakten Zellwand in die Zellen des Zielgewebes, sondern auch direkt über die temporär entstandenen Poren in dem Zielgewebe als Aufnahmeweg in die Zellen einwandern.

Als vorteilhaft erweist sich auch, dass die flexible Struktur der Beschichtung, insbesondere bei der Verwendung von Microbubbles, ein hohes plastisches Verformungsvermögen aufweist. Dadurch bleibt die Integrität der Beschichtung auch während Prozessierung und Implantation des Stents erhalten. Vor der gezielten Zerstörung der Beschichtung erfolgt daher keine nennenswerte Delamination der Beschichtung.

Der bevorzugte Bereich der Resonanzfrequenz, der zwischen 0,2 MHz und 10 MHz liegt, richtet sich für jeden Implantattyp nach seinem bestimmungsgemäßen Verwendungsort, und zwar durch die grundlegende Bedingung, dass die Reichweite des Ultraschalls in diesem Frequenzbereich so groß sein muss, dass in der Tiefe des Implantationsorts unter der Körperoberfläche noch die erforderliche Schallintensität erreicht wird, die ausreicht, um die Wandungen der Hohlräume derart zum Schwingen anzuregen, dass diese reißen oder brechen. Für koronare Implantate beispielsweise beträgt die Implantationstiefe ca. 10 cm bis 20 cm, wodurch der bevorzugte Resonanzbereich im Frequenzbereich zwischen 0,2 MHz und 2 MHz liegt, weiter bevorzugt zwischen 0,2 MHz und 1 MHz. Für periphere Gefäßstützen im Bein- oder Halsbereich mit Tiefen von wenigen Zentimetern kommen höhere Frequenzen in Betracht, die vorzugsweise zwischen 1 MHz und 10 MHz, besonders bevorzugt zwischen 2 MHz und 5 MHz liegen. Resonanzfrequenzen in dem bestimmten Bereich lassen sich durch geeignete Wahl des Materials, der Dicke der Wandung und der mittleren Größe der Hohlräume erreichen. Die für die Anwendung maßgebliche Resonanzfrequenz ist eine Funktion des Hohlraumdurchmessers, der chemischen Zusammensetzung der Wandung der Hohlräume und der Charakteristik der Wechselwirkungen zwischen den Hohlräumen in den erfindungsgemäßen Beschichtungen. Der mittlere Hohlraumdurchmesser liegt dabei vorzugsweise im Bereich von 50 nm bis 25 µm, besonders bevorzugt im Bereich von 50 nm bis 5 µm.

Im Rahmen der vorliegenden Erfindung wird mit der Resonanzfrequenz die Frequenz gemeint, bei der das Gesamtsystem der Hohlräume in wässriger Umgebung bzw. in der Umgebung der Körperflüssigkeit eine Resonanz aufweist. Als Testumgebung hierfür kann beispielsweise eine 0,9%ige Kochsalzlösung dienen.

Um den Abrieb der Beschichtung bei dem Transport des Implantats zum Ort der Behandlung im Körper des behandelten Organismus zu vermeiden, kann die Beschichtung vorzugsweise in geschützten Teilen der tragenden Struktur des Implantatkörpers angeordnet werden, vorzugsweise auf der luminalen Seite des Implantatkörpers und/oder in Ausnehmungen (Kehlen) und/oder in Vertiefungen und/oder in lochähnlichen Durchbrüchen des Implantatkörpers. Vorzugsweise erfolgt das Aufbringen der Beschichtung mit den Hohlräumen, vorzugsweise mit den Microbubbles, mittels einer wässrigen Suspension. Gegebenenfalls können andere Lösemittel, z.B. Dimethylsulfoxid (DMSO), eingesetzt werden.

In einer weiteren Ausführungsform des erfindungsgemäßen Implantats weist dieses eine Beschichtung mit einer ersten Schicht umfassend ausschließlich ungefüllte Hohlräume und eine zweiten Schicht enthaltend mindestens eine pharmazeutisch aktive Substanz und/oder mindestens eine diagnostische Substanz auf, wobei entweder zuerst die erste Schicht auf die Oberfläche des Implantatkörpers und danach die zweite Schicht auf die Oberfläche der ersten Schicht aufgebracht wird oder die Beschichtung in umgekehrter Reihenfolge erfolgt. Der Vorteil der ersten Variante der Schichtstruktur mit einer unter der zweiten Schicht angeordneten ersten Schicht besteht darin, dass eine Wirkstoffabgabe aus der zweiten Schicht nicht durch die Eigenschaften der ersten Schicht, z.B. hinsichtlich Diffusion, beeinflusst wird. Der Vorteil der zweiten Variante der Schichtstruktur, bei der die erste Schicht oberhalb der zweiten Schicht angeordnet ist, besteht darin, dass die Wirkstoffabgabe aus der zweiten Schicht durch die Zerstörung der Hohlraumschicht (erste Schicht) gesteuert werden kann, vorzugsweise durch die oben beschriebene Anregung mittels einer Ultraschallquelle von einem Ort außerhalb des Körpers.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, der oder das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiphlogistika (wie beispielsweise Cortison oder Diclofenac), der Antiinflammatorica (wie beispielsweise Imidazol), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel, oder Sirolimus und Derivate (Rapaloge), sowie der Epothilone bestehen. Weitere pharmazeutisch aktive Substanzen umfassen Immunmodulatoren, Statine, kardiovaskuläre Wirkstoffe, Nukleinsäuren wie Plasmide, siRNA, miRNA und dsRNA.

Zu den diagnostischen Substanzen im Sinne der Erfindung zählen unter anderem Kontrastmittel für Röntgen- oder MR-Diagnostik (z.B. seltene Erden) und spezifische Substanzen zur gezielten Markierung bestimmter körpereigener Strukturen, einschließlich bestimmter Zelltypen, zellulärer Bestandteile, Botenstoffe und/oder Biomarker.

Die Degradation der Beschichtung und somit des Implantats kann noch besser gesteuert werden, wenn die Hülle der Hohlräume biodegradierbare Materialien enthält, vorzugsweise mindestens ein Material der Gruppe enthaltend Lipide, Phospholipide, Polysaccharide, Proteine, Peptide, Kollage, Elastine, Fibrine, Chitosane, Hyaluronsäuren, Chondroitinsulfate, Hydrogele und synthetische Polymere, beispielsweise PLA, Polyamidester, Polyvinylester, Polyvinylalkohole.

Für den Implantatkörper oder die Beschichtung geeignete biodegradierbare Werkstoffe können beispielsweise Polymere oder Metalle enthalten. Der Körper oder die Beschichtung können dabei aus mehreren dieser Werkstoffe bestehen. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephthalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen.

Werden Hohlräume in Form von Mikrokapseln oder Microbubbles verwendet, so können diese mittels einer einbettenden Matrix enthaltend mindestens ein Material der Gruppe enthaltend Polymere, Lipide, Weichmacher auf Zitratbasis (z.B. BTHC (n-Butyryl-tri-n-hexylcitrat)), Proteine und Peptide und/oder mittels eines Haftvermittlers und/oder mittels auf der Oberfläche der Microbubbles oder Mikrokapseln angeordneten Ankergruppen auf der Oberfläche des Implantatkörpers befestigt sein. Weitere Alternativen beziehungsweise zusätzliche Möglichkeiten der Befestigung der Microbubbles oder Mikrokapseln auf der Oberfläche des Implantatkörpers bestehen darin, im Verband einen Formschluss zu erreichen, aufgrund chemischer Wechselwirkungen der Bestandteile der Einbettungsmatrix, z.B. Proteinen, oder aufgrund chemischer Wechselwirkung der Wandbestandteile der Hohlräume, vorzugsweise Proteine, Peptide oder Substanzen mit analogen chemischen Eigenschaften. In einem Ausführungsbeispiel kann das Matrixmaterial zusätzlich eine pharmazeutisch aktive Substanz und/oder diagnostische Substanz aufweisen.

Die Beschichtung mit den Hohlräumen, vorzugsweise mit den Microbubbles, wird mittels Tauchen, Pipettieren oder Sprühen, vorzugsweise als wässrige Suspension, gegebenenfalls mit anschließender Trocknung, hergestellt. Vor, während oder nach dem Aufbringen der Hohlräume, vorzugsweise der Microbubbles, z.B. als wässrige Suspension kann die einbettenden Matrix, gegebenenfalls in einer wässrigen Lösung, enthaltend mindestens ein Material der Gruppe enthaltend Polymere, Lipide, Weichmacher auf Zitratbasis, Proteine und Peptide und/oder ein Haftvermittler auf die entsprechenden Bereiche des Implantatkörpers aufgebracht werden, vorzugsweise ebenfalls mittels Tauchen, Pipettieren oder Sprühen. Falls gewünscht, kann noch eine weitere, z. B. polymere Schicht auf die Beschichtung mit den Hohlräumen als Korrosionsschutz aufgebracht werden, um die Degradation vor der ultraschallaktivierten Zerstörung der Wandungen der Hohlräume zu minimieren oder die Gleitfähigkeit der Beschichtung zu erhöhen. Geeignete Materialien der weiteren Schicht sind bevorzugt die zuvor bereits genannten biodegradierbaren Polymere. Aufgrund der unregelmäßigen Struktur der Beschichtung ergibt sich eine gute Haftung der weiteren Schicht auf der Beschichtung.
Die obige Aufgabenstellung wird ferner gelöst durch ein Implantat erhältlich oder erhalten durch eines der oben beschriebenen erfindungsgemäßen Verfahren. Ein derartiges Implantat weist die oben im Zusammenhang mit dem erfindungsgemäßen Herstellungsverfahren angegebenen Vorteile auf.
Das erfindungsgemäße Verfahren bzw. das erfindungsgemäße Implantat werden nachfolgend in Beispielen anhand von Figuren erläutert. Dabei bilden alle beschriebenen und/oder bildlich dargestellten Merkmale den Gegenstand der Erfindung. Es zeigen schematisch jeweils in einem Querschnitt:
- Fig. 1: ein erstes Ausführungsbeispiel eines erfindungsgemäßen Implantats,
- Fig. 2: ein zweites Ausführungsbeispiel eines erfindungsgemäßen Implantats,
- Fig. 3: ein drittes Ausführungsbeispiel eines erfindungsgemäßen Implantats,
- Fig. 4: ein viertes Ausführungsbeispiel eines erfindungsgemäßen Implantats und
- Fig. 5: ein fünftes Ausführungsbeispiel eines erfindungsgemäßen Implantats.

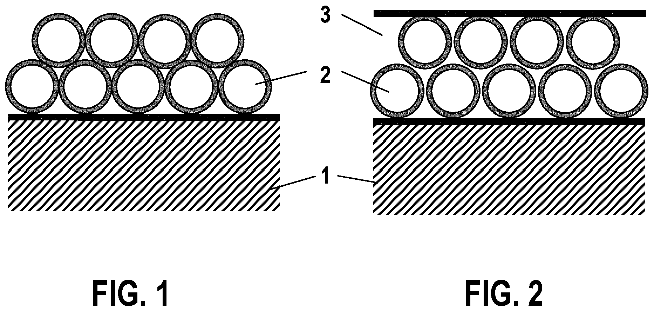

Figur 1 zeigt einen Abschnitt eines Stents, nämlich den Abschnitt einer Stentstrebe 1 bestehend aus einer Magnesiumlegierung mit einem Anteil von mindestens 50 Gew.% Magnesium. Auf der äußeren Oberfläche der Stentstrebe 1 ist eine Beschichtung mit Microbubbles 2 angeordnet, die aus PLA besteht. Der Innenraum der Microbubbles 2 ist mit einem inerten, schwer löslichen Gas, z.B. Stickstoff oder Schwefelhexafluorid (SF₆), gefüllt. Der mittlere Durchmesser der Microbubbles 2 beträgt etwa 5 µm. Die Microbubbles 2 werden durch ihre Oberflächenspannung auf der äußeren Oberfläche der Stentstrebe 1 befestigt, zusätzlich können chemische Ankerketten auf/in die Wand der Microbubbles 2 eingebracht sein, um die Haftung der Microbubbles 2 auf der Oberfläche der Stentstrebe 1 zu verbessern.

Das in Figur 2 dargestellte zweite Ausführungsbeispiel entspricht dem Beispiel, das in Figur 1 gezeigt ist, mit dem Unterschied, dass die Microbubbles 2 in eine Matrix 3 aus PLLA oder Hyaluronsäure eingebettet sind.

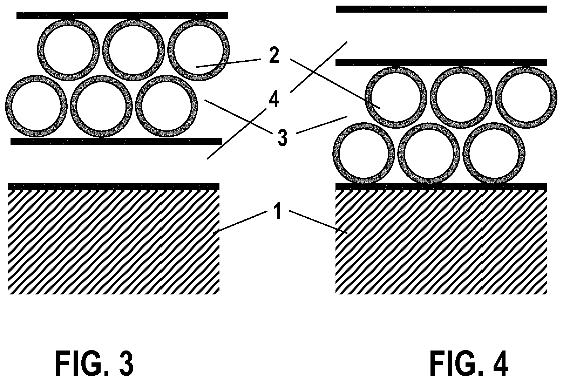

Anhand der Figuren 3 und 4 werden Ausführungsbeispiele eines erfindungsgemäßen Implantats dargestellt, die dem zweiten Ausführungsbeispiel entsprechen und zusätzlich eine Schicht 4 mit einer pharmazeutisch aktiven Substanz aufweisen, wobei die Schicht 4 beispielsweise im Fall der Figur 3 BTHC und Paclitaxel und im Fall der Figur 4 PLLA und Sirolimus enthält. In dem in Figur 3 gezeigten dritten Ausführungsbeispiel ist die Schicht 4 unterhalb der Schicht mit den Microbubbles 2 angeordnet, während bei dem in Figur 4 dargestellten Ausführungsbeispiel die Schicht 4 mit der pharmazeutisch aktiven Substanz oberhalb der Schicht mit den Microbubbles 2 liegt. Analog zu dem zweiten Ausführungbeispiel sind die Microbubbles 2 jeweils in die Matrix 3 eingebettet. Alternativ können die Microbubbles analog zu dem in Figur 1 gezeigten ersten Ausführungsbeispiel jeweils auch ohne Matrixmaterial auf der Oberfläche der Stentstrebe 1 oder der Schicht 4 befestigt sein.

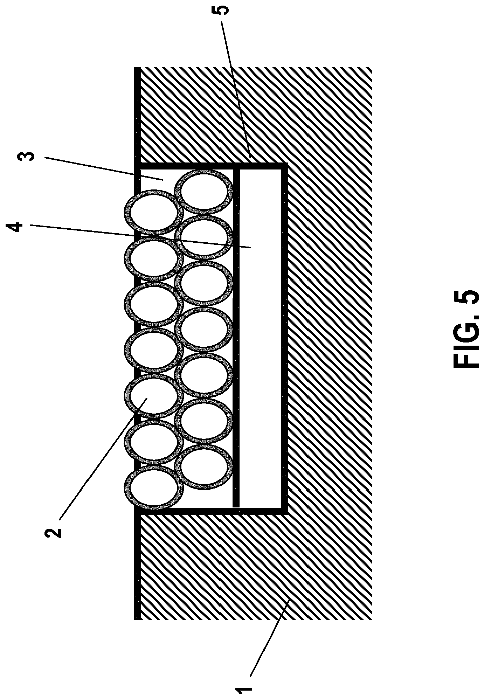

Schließlich zeigt Figur 5 ein Ausführungsbeispiel, bei dem eine Schicht 4 mit Hyaluronsäure und einem Entzündungshemmer in einer Ausnehmung 5 in der Stentstrebe 1 angeordnet ist. Über der Schicht 4 liegt, ebenfalls in der Ausnehmung 5, eine weitere Schicht mit Microbubbles 2 bestehend aus PLA mit einer Matrix 3 aus PLLA oder Hyaluronsäure, die auf der Oberfläche der Schicht 4 und innerhalb der Ausnehmung 5 angeordnet ist. Alternativ können die Microbubbles 2 auf der Schicht 4 in der Ausnehmung 5 aufgrund ihrer Oberflächenspannung oder durch Formschluss in der Ausnehmung 5, d.h. ohne Matrix, angeordnet sein.

Die Herstellung eines erfindungsgemäßen Implantats wird im Folgenden am Beispiel einer biodegradierbaren Gefäßstütze in Form eines Koronarstents dargestellt.

Als Grundkörper der Gefäßstütze dient ein biodegradierbarer, ballonexpandierbarer Metallstent, hergestellt mittels Laserschneiden aus einem Röhrchen aus der biokompatiblen, biodegradierbaren Magnesiumlegierung WE43. Der Stent wird an mehreren definierten Stellen mit Vertiefungen zur Aufnahme von Microbubbles versehen. Die Vertiefungen können ebenfalls mittels Laserschneiden hergestellt werden. Die Vertiefungen werden bevorzugt in Stegbereichen angeordnet, die beim Dilatieren weniger stark mechanisch beansprucht oder gar verformt werden als andere Bereiche.

Um eine Beschichtung gezielt in die Vertiefungen einbringen zu können, wird der Stent in einer Mikropipettieranlage auf eine Positioniervorrichtung montiert.

Unmittelbar vor der Beschichtung wird eine Microbubble-Suspension von 2 µm bis 10 µm großen Mikrobläschen mit einer Phospholipid-Hülle enthaltend das Füllgas SF₆ präpariert.

Die Rekonstitution der Suspension aus dem Granulat (z.B. SonoVue® der Fa. BRACCO Imaging S.p.A., Amsterdam) erfolgt vorzugsweise unter strengem Luftabschluss, unter Zugabe eines wässrigen Lösemittels, beispielsweise 0,9%ige Kochsalzlösung (ggf. mit Zusatzstoffen), und intensivem Schütteln mindestens über einen Zeitraum von 20 s bis eine homogene, milchig weiße Suspension entsteht. Die so hergestellte Suspension wird in die Mikropipettieranlage gefüllt und auf/in die oben erwähnten Vertiefungen oder andere geschützte Bereiche auf den Grundkörper aufgebracht. Die deponierten Tröpfchen der Suspension mit den Microbubbles bleiben durch ihre Oberflächenspannung in den Vertiefungen haften. In einem bevorzugten Ausführungsbeispiel wird der Vortrieb der Suspension in der Mikropipettieranlage durch Druckbeaufschlagung mit dem Füllgas SF₆ bewirkt. Ferner dient dieses Gas dazu, das Eindringen anderer Gase zu verhindern.

Alternativ oder zusätzlich zu Phospholipiden können die Microbubble-Hüllen mindestens ein Material aus der Gruppe umfassend Galaktose, Albumin und Perflutren enthalten.

Im nächsten Schritt wird die Matrixkomponente, hier Hyaluronsäure in wässriger Lösung, zu der in den Vertiefungen angeordneten Schicht mit den Microbubbles in gleicher Weise mittels der Mikropipettieranlage zugegeben. Die Matrixkomponente dient zur Anhaftung der Microbubbles an der Oberfläche des Stents. Durch die nachträgliche Aufbringung kommt die Matrixkomponente vorwiegend in den Außenbereichen der Beschichtung zu liegen und kann so die tiefer liegenden Bubbles besser vor Beschädigung schützen.

Alternativ kann die Matrixkomponente der Schicht, hier Hyaluronsäure in wässriger Lösung, mittels der Mikropipettieranlage zusammen (d.h. zeitgleich) mit der Microbubble-Suspension in die Vertiefungen aufgetragen werden. Hierfür werden die beiden Lösungen unmittelbar vor dem Austreten aus der Austrittsöffnung der Mikropipettieranlage aus getrennten Reservoirs zusammengeführt. Die Matrixkomponente verteilt sich dabei gleichmäßig in der Beschichtung und führt so zu gleichmäßigen akustischen Eigenschaften der eingebetteten Microbubbles. Durch einen Zeitversatz beim Abschalten der treibenden Pumpen kann erreicht werden, dass in den Außenbereichen der Beschichtung überwiegend die Matrixkomponente angeordnet ist, so dass die tiefer liegenden Microbubbles vor Beschädigung geschützt werden.

Anschließend wird bei beiden Varianten der Aufbringung ein (unvollständiger / partieller) Trocknungsschritt durchgeführt, der vor allem den Zweck hat, die äußere Oberfläche der in den Vertiefungen angeordneten Bubble-Depots für die nachfolgenden Beschichtungsschritte zu stabilisieren. Der beschichtete Stent-Grundkörper wird dazu in einer trockenen Umgebung, z.B. in einer langsamen (laminaren) Strömung von kaltem oder moderat erwärmtem Stickstoff oder Argon oder einem anderen inerten Gas, z.B. bei einer Temperatur im Bereich von 10°C bis 50°C, über einen vorgegebenen Zeitraum von wenigen Minuten ausgelagert.

Alternativ kann für das Trocknen statt der langsamen Strömung ein geschlossener Trockenraum mit Wasserentzug durch mindestens einen Kühlfinger (gekühlt mit Flüssigstickstoff, Trockeneis oder einer Peltierkühlung) oder ein Temperofen mit stehender InertgasAtmosphäre verwendet werden.

Schließlich wird der Stent voll- oder teilflächig mit einer Beschichtung enthaltend PLLA versehen, die mittels Sprühbeschichtung aufgetragen wird. Die PLLA-Beschichtung stellt einen Korrosionsschutz für die entsprechenden Bereiche des Stents dar, verbessert die mechanische Haftung der Microbubble-Schicht und schützt sie gegen Abrieb.

### Bezugszeichenliste

- 1: Stentstrebe
- 2: Microbubble
- 3: Matrix
- 4: Schicht mit pharmazeutisch aktiver Substanz
- 5: Ausnehmung

## Patentansprüche

1. Verfahren zur Herstellung eines degradierbaren Implantats, insbesondere einer degradierbaren intraluminalen Endoprothese, mit einem Körper, der zumindest auf einem Teil seiner Oberfläche eine Beschichtung aufweist, umfassend die folgenden Schritte:
a) Bereitstellen des Implantatkörpers,
b) Aufbringen der Beschichtung auf die Oberfläche des Implantatkörpers, wobei die Beschichtung ungefüllte Hohlräume, vorzugsweise in Form von Microbubbles (2), aufweist.

2. Verfahren nach Anspruch 1, **dadurch gekennzeichnet, dass** die Hohlräume eine Ultraschall-Resonanzfrequenz aufweisen, die zwischen etwa 0,2 MHz und etwa 10 MHz liegt.

3. Verfahren nach einem der vorhergehenden Ansprüche, **dadurch gekennzeichnet, dass** die Hülle der Hohlräume biodegradierbare Materialien enthält, vorzugsweise mindestens ein Material der Gruppe enthaltend synthetische Polymere, beispielsweise PLA, Polyamidester, Polyvinylester, Polyvenylalkohole, weiter enthaltend Lipide, Polysaccharide, Proteine, Peptide, Kollagene, Elastine, Fibrine, Chitosane, Hyaluronsäuren, Chondroitinsulfate und Hydrogele.

4. Verfahren nach einem der vorhergehenden Ansprüche, **dadurch gekennzeichnet, dass** die durch die Beschichtung gebildete Umhüllung der Hohlräume oder die Hülle der Microbubbles (2) mittels Ultraschall zerstörbar ist.

5. Verfahren nach einem der vorhergehenden Ansprüche, **dadurch gekennzeichnet, dass** die Hohlräume, vorzugsweise die Microbubbles, mittels einer wässrigen Suspension, z.B. auf der luminalen Seite der Stege (1) des Implantatkörpers und/oder in Ausnehmungen (5) und/oder in Vertiefungen und/oder in lochähnlichen Durchbrüchen des Implantatkörpers angeordnet werden.

6. Verfahren nach einem der vorhergehenden Ansprüche, **dadurch gekennzeichnet, dass** vor, während oder nach dem Aufbringen der Hohlräume, vorzugsweise der Microbubbles (2), eine einbettende Matrix (3), enthaltend mindestens ein Material der Gruppe enthaltend Polymere, Lipide, Weichmacher auf Zitratbasis, Proteine und Peptide und/oder ein Haftvermittler auf die entsprechenden Bereiche des Implantatkörpers aufgebracht wird.

7. Verfahren nach einem der vorhergehenden Ansprüche, **dadurch gekennzeichnet, dass** die Beschichtung mindestens zwei Schichten aufweist, vorzugsweise eine erste Schicht umfassend ausschließlich ungefüllte Hohlräume und eine zweite Schicht (4) enthaltend mindestens eine pharmazeutisch aktive Substanz und/oder mindestens eine diagnostische Substanz und/oder einen Korrosionsschutz.

8. Degradierbares Implantat, insbesondere intraluminale Endoprothese, mit einem Körper, der zumindest auf einem Teil seiner Oberfläche eine Beschichtung aufweist, **dadurch gekennzeichnet, dass** die Beschichtung ungefüllte Hohlräume, vorzugsweise in Form von Microbubbles (2), aufweist.

9. Implantat nach einem der Ansprüche 8, **dadurch gekennzeichnet, dass** die Resonanzfrequenz der Hohlräume zwischen etwa 0,2 MHz und etwa 10 MHz liegt.

10. Implantat nach einem der Ansprüche 8 bis 9, **dadurch gekennzeichnet, dass** die Umhüllung der Hohlräume biodegradierbare Materialien enthält, vorzugsweise mindestens ein Material der Gruppe enthaltend Lipide, Phospholipide, Polysaccharide, Proteine, Peptide, Kollagene, Elastine, Fibrine, Chitosane, Hyaluronsäuren, Chondroitinsulfate, Hydrogele und synthetische Polymere, beispielsweise PLA, Polyamidester, Polyvinylester, Polyvinylalkohole.

11. Implantat nach einem der Ansprüche 8 bis 10, **dadurch gekennzeichnet, dass** die durch die Beschichtung gebildete Umhüllung der Hohlräume oder die Hülle der Microbubbles (2) mittels Ultraschall zerstörbar ist.

12. Implantat nach einem der Ansprüche 8 bis 11, **dadurch gekennzeichnet, dass** die Beschichtung mindestens zwei Schichten aufweist, vorzugsweise eine erste Schicht enthaltend ausschließlich ungefüllte Hohlräume und eine zweite Schicht (4) enthaltend mindestens eine pharmazeutisch aktive Substanz und/oder mindestens eine diagnostische Substanz und/oder einen Korrosionsschutz.

13. Implantat nach einem der Ansprüche 8 bis 12, **dadurch gekennzeichnet, dass** die Beschichtung auf der luminalen Seite der Stege (1) des Implantatkörpers und/oder in Ausnehmungen (5) und/oder in Vertiefungen und/oder in lochähnlichen Durchbrüchen angeordnet ist.

14. Implantat nach einem der Ansprüche 8 bis 13, **dadurch gekennzeichnet, dass** die Microbubbles (2) mittels einer einbettenden Matrix (3) enthaltend mindestens ein Material der Gruppe enthaltend Polymere, Lipide, Weichmacher auf Zitratbasis, Proteine und Peptide und/oder mittels eines Haftvermittlers und/oder mittels auf der Oberfläche der Microbubbles (2) angeordneten Ankergruppen auf der Oberfläche des Implantatkörpers befestigt sind.

## Claims

1. A method for manufacturing a degradable implant, in particular a degradable intraluminal endoprosthesis, comprising a body having a coating on at least a portion of the surface thereof, said method comprising the following steps:
a) providing the implant body,
b) applying the coating to the surface of the implant body, wherein the coating comprises unfilled cavities, preferably in the form of microbubbles (2).

2. The method according to claim 1, **characterised in that** the cavities have an ultrasound resonance frequency which is between 0.2 MHz and approximately 10 MHz.

3. The method according to either one of the preceding claims, **characterised in that** shell of the cavities contains biodegradable materials, preferably at least one material selected from the group containing synthetic polymers, for example PLA, polyamide esters, polyvinyl esters, polyvinyl alcohols, also containing lipids, polysaccharides, proteins, peptides, collagens, elastins, fibrins, chitosans, hyaluronic acids, chondroitin sulphates, and hydrogels.

4. The method according to any one of the preceding claims, **characterised in that** the covering of the cavities formed by the coating, or a shell of the microbubbles (2) can be destroyed using ultrasound.

5. The method according to any one of the preceding claims, **characterised in that** the cavities, preferably the microbubbles, are disposed by way of an aqueous suspension for example on the luminal side of the struts (1) of the implant body and/or in recesses (5) and/or in indentations and/or in hole-like openings of the implant body.

6. The method according to any one of the preceding claims, **characterised in that** before, during or after the application of the cavities, preferably the microbubbles (2), an embedding matrix (3) containing at least one material selected from the group containing polymers, lipids, citrate-based softening agents, proteins and peptides and/or an adhesion promoter is applied to the corresponding regions of the implant body.

7. The method according to any one of the preceding claims, **characterised in that** the coating has at least two layers, preferably a first layer comprising only unfilled cavities, and a second layer (4) containing at least one pharmaceutically active substance and/or at least one diagnostic substance and/or corrosion protection.

8. A degradable implant, in particular an intraluminal endoprosthesis, comprising a body which has a coating on at least a portion of the surface thereof, **characterised in that** the coating comprises unfilled cavities, preferably in the form of microbubbles (2).

9. The implant according to claim 8, **characterised in that** the resonance frequency of the cavities is between approximately 0.2 MHz and approximately 10 MHz.

10. The implant according to either one of claims 8 or 9, **characterised in that** the covering of the cavities contains biodegradable materials, preferably at least one material from the group containing lipids, phospholipids, polysaccharides, proteins, peptides, collagens, elastins, fibrins, chitosans, hyaluronic acids, chondroitin sulphates, hydrogels and synthetic polymers, for example PLA, a polyamide esters, polyvinyl esters, polyvinyl alcohols.

11. The implant according to any one of claims 8 to 10, **characterised in that** the covering of the cavities formed by the coating, or the shell of the microbubbles (2) can be destroyed using ultrasound.

12. The implant according to any one of claims 8 to 11, **characterised in that** the coating has at least two layers, preferably a first layer containing only unfilled cavities, and a second layer (4) containing a least one pharmaceutically active substance and/or at least one diagnostic substance and/or corrosion protection.

13. The implant according to any one of claims 8 to 12, **characterised in that** the coating is disposed on the luminal side of the struts (1) of the implant body and/or in recesses (5) and/or in indentations and/or in hole-like openings.

14. The implant according to any one of claims 8 to 13, **characterised in that** the microbubbles (2) are attached to the surface of the implant body using an embedding matrix (3) containing at least one material from the group containing polymers, lipids, citrate-based softening agents, proteins and peptides and/or using an adhesion promoter and/or using anchor groups disposed on the surface of the microbubbles (2).

## Revendications

1. Procédé de fabrication d'un implant dégradable, notamment une endoprothèse intraluminale dégradable, doté d'un corps qui présente un revêtement sur au moins une partie de sa surface, comprenant les étapes suivantes :
a) mise au point du corps d'implant,
b) dépôt du revêtement sur la surface du corps d'implant, où le revêtement présente des cavités, de préférence sous la forme de microbulles (2).

2. Procédé selon la revendication 1, **caractérisé en ce que** les cavités présentent une fréquence de résonance aux ultrasons qui se situe entre environ 0,2 MHz et environ 10 MHz.

3. Procédé selon l'une des revendications précédentes, **caractérisé en ce que** l'enveloppe des cavités contient des matériaux biodégradables, de préférence, au moins un matériau du groupe constitué de polymères synthétiques, par exemple, d'acide polylactique PLA, de polyamides esters, d'esters de polyvinyle, d'alcools de polyvinyle, contenant en outre des lipides, des polysaccharides, des protéines, des peptides, des collagènes, de l'élastine, de la fibrine, du chitosan, des acides hyaluroniques, des sulfates de chondroitine et des hydrogels.

4. Procédé selon l'une des revendications précédentes, **caractérisé en ce que** l'enveloppe formée par le revêtement des cavités ou l'enveloppe des microbulles (2) peuvent être détruites au moyen d'ultrasons.

5. Procédé selon l'une des revendications précédentes, **caractérisé en ce que** les cavités, de préférence, les microbulles, sont disposées au moyen d'une suspension aqueuse, par exemple, sur la face luminale des tiges (1) du corps d'implant et/ou dans des évidements (5) et/ou des renfoncements, et/ou dans des interruptions semblables à des trous du corps d'implant.

6. Procédé selon l'une des revendications précédentes, **caractérisé en ce qu'**avant, pendant ou après la réalisation des cavités, de préférence, des microbulles (2), une matrice (3) d'ensevelissement contenant au moins un matériau du groupe constitué de polymères, de lipides, de plastifiants à base de citrate, de protéines et de peptides, et/ou un promoteur d'adhésion est rapportée sur les zones correspondantes du corps d'implant.

7. Procédé selon l'une des revendications précédentes, **caractérisé en ce que** le revêtement présente au moins deux couches, de préférence, une première couche comprenant exclusivement des cavités non remplies et une deuxième couche (4) contenant au moins une substance pharmaceutiquement active, et/ou au moins une substance de diagnostic, et/ou une protection anticorrosion.

8. Implant dégradable, notamment endoprothèse intraluminale, dotée d'un corps qui présente un revêtement sur au moins une partie de sa surface, **caractérisé en ce que** le revêtement présente des cavités non remplies, de préférence, sous forme de microbulles (2).

9. Implant selon l'une des revendications 8, **caractérisé en ce que** la fréquence de résonance des cavités se situe entre environ 0,2 MHz et environ 10 MHz.

10. Implant selon l'une des revendications 8 à 9, **caractérisé en ce que** l'enveloppe des cavités contient des matériaux biodégradables, de préférence au moins un matériau du groupe constitué des lipides, des phospholipides, des polysaccharides, des protéines, des peptides, des collagènes, de l'élastine, de la fibrine, du chitosan, des acides hyaluroniques, des sulfates de chondroitine, des hydrogels et des polymères synthétiques, par exemple, de l'acide polylactique, des polyamide esters, des esters de polyvinyle, des alcools polyvinyliques.

11. Implant selon l'une des revendications 8 à 10, **caractérisé en ce que** l'enveloppe des cavités formée par le revêtement ou l'enveloppe des microbulles (2) peut être détruite au moyen d'ultrasons.

12. Implant selon l'une des revendications 8 à 11, **caractérisé en ce que** le revêtement présente au moins deux couches, de préférence une première couche contenant exclusivement des cavités et une deuxième couche (4) contenant au moins une substance pharmaceutiquement active, et/ou au moins une substance de diagnostic, et/ou une protection anticorrosion.

13. Implant selon l'une des revendications 8 à 12, **caractérisé en ce que** le revêtement est disposé sur la face luminale des tiges (1) dans le corps d'implant, et/ou dans des évidements (5), et/ou des renfoncements, et/ou dans des interruptions semblables à des trous.

14. Implant selon l'une des revendications 8 à 13, **caractérisé en ce que** les microbulles (2) sont fixées sur la surface du corps d'implant au moyen d'une matrice (3) d'ensevelissement contenant au moins un matériau du groupe constitué de polymères, de lipides, de plastifiants à base de citrate, de protéines et de peptides, et/ou au moyen d'un promoteur d'adhésion, et/ou au moyen de groupes d'ancrages disposés sur la surface des microbulles (2).
